# EUROPÄISCHE PATENTSCHRIFT

(11) **EP 0 285 972 B1**
(45) Veröffentlichungstag und Bekanntmachung des Hinweises auf die Patenterteilung: **11.08.1993**
(21) Anmeldenummer: 88105032.2
(22) Anmeldetag: 29.03.1988
(51) Int. Cl.: C07C 309/46

(54) **Verfahren zur Herstellung von 2,4-Diaminobenzolsulfonsäure**
Process for the preparation of 2,4-diaminobenzenesulfonic acid
Procédé de préparation de l'acide 2,4-diaminobenzène sulfonique

(30) Priorität: 08.04.1987 DE 3711835
(43) Veröffentlichungstag der Anmeldung: 12.10.1988
(73) Patentinhaber: BASF Aktiengesellschaft, 67063 Ludwigshafen (DE)
(72) Erfinder: Lardon, Hartmut, Dr., D-6730 Neustadt (DE); Hackenberger Alfred, Dr., BR-09700 Sao Bernardo do Campo-Sp. (BR); Patsch, Manfred, Dr., D-6706 Wachenheim (DE)

(56) Entgegenhaltungen:
- EP-A- 0 146 889
- DE-C- 65 240
- GB-A- 1 517 871
- CHEMICAL ABSTRACTS, Band 102, Nr. 7, 18. Februar 1985, Seite 566, Zusammenfassung Nr. 61884n, Columbus, Ohio, US; K. Filipiak et al.: "Catalytik reduction of 2,4-dinitrobenzenesulfonic acid"

## Beschreibung

Die vorliegende Erfindung betrifft ein verbessertes Verfahren zur Herstellung von Metaminsäure
Nach bekannten Verfahren wird Metaminsäure durch Sulfonierung von m-Phenylendiamin, Oxidation von Di-(o,p-diaminophenyl)-disulfid oder durch Reduktion von 2,4-Dinitrobenzolsulfonsäure, z.B. durch Béchamp-Reduktion (s. Houben-Weyl, Methoden der Organischen Chemie, Bd. XI/1, S. 394) hergestellt. Das letztgenannten Verfahren besitzt jedoch eine Reihe von Nachteilen: die Abtrennung des Endstoffs vom Eisenoxidschlamm ist zeitaufwendig und führt zu Ausbeuteminderungen durch Anteile, die im Eisenoxidschlamm verbleiben. Außerdem fallen größere Mengen an Natriumacetat zwangsmäßig an, da die Reduktion im allgemeinen in essigsaurer Lösung bei einem pH-Wert von 3 durchgeführt wird.

Versuche, die Béchamp-Reduktion durch die katalytische Hydrierung mit Raney-Nickel als Katalysator zu ersetzen, hatten häufig nicht den gewünschten Erfolg: die Wasserstoffaufnahme erfolgt nicht quantitativ, da der Katalysator durch schwefelhaltige Verbindungen vergiftet wird. Um einen vollständigen Umsatz zu erreichen, muß bei höheren Drücken (20 -200 bar) gearbeitet werden, was den Einsatz teurer Hochdruckapparate erforderlich macht. Gemäß der GB-PS 1 517 871 müssen Nitrobenzolsulfonsäuren vor der Hydrierung gereinigt werden, um eine Vergiftung des Katalysators zu vermeiden. Solche aufwendigen Reinigungsoperationen können vermieden werden, wenn man die dort beschriebene, technisch aufwendige Reaktionsführung einhält und die Reduktion bei einem Druck von bis zu 40 bar, vorteilhaft 10 bis 20 bar und einer Temperatur von 70 bis 150°C durchführt.

Um bei niedrigeren Drücken eine weitgehende Hydrierung von Nitrobenzolsulfonsäuren zu bewerkstelligen, wurde vorgeschlagen, die Hydrierung in Gegenwart von Promotoren wie Borsäure oder Borsäure abgebender Mittel (DE-OS 23 01 739) oder vorteilhaft in Gegenwart eines Gemisches aus
a) 1 bis 25 Gew.% Borsäure und/oder Borat und
b) 0,1 bis 0,9 Gew.% Phosphorsäure und/oder Phosphat,
bezogen auf den Ausgangsstoff durchzuführen (DE-OS 33 47 452). Diese Verfahren sind jedoch alle im Falle von zuvor gereinigten Nitrobenzolsulfonsäuren angewandt worden.

Der Erfindung lag nun die Aufgabe zugrunde, ein einfaches und wirtschaftliches Verfahren zur Herstellung von Metaminsäure zu finden, wobei insbesondere aufwendige Isolierungs- und Reinigungsoperationen des Zwischenproduktes 2,4-Dinitrochlorbenzol vermieden werden sollten, ohne daß merkliche Ausbeuteeinbußen bei der anschließenden Reduktion auftreten.

Demgemäß wurde ein Verfahren zur Herstellung von Metaminsäure gefunden, das dadurch gekennzeichnet ist, daß man in einer ersten Stufe 2,4-Dinitrochlorbenzol in wäßrigem Medium in Gegenwart
a) eines organischen Lösungs- oder Verdünnungsmittels und/oder
b) katalytischer Mengen eines ionischen oder nichtionischen Tensids oder Dispergiermittels
bei einem pH von 6 bis 8 mit Salzen der schwefligen Säure umsetzt, das Reaktionsgemisch ansäuert und die so gebildete 2,4-Dinitrobenzolsulfonsäure in situ katalytisch bei Atmosphärendruck oder Drücken von 1 bis 9 bar in Gegenwart eines Promotors hydriert.

In der ersten Verfahrensstufe wird 2,4-Dinitrochlorbenzol in Wasser suspendiert und mit einem sauren oder vorzugsweise neutralen Salz der schwefligen Säure, vorteilhaft einem Alkali- oder Ammoniumsalz, insbesondere mit Natriumhydrogensulfit oder Natriumsulfit umgesetzt, wobei man zweckmäßigerweise 1 bis 2 Äquivalente, bevorzugt 1,2 bis 1,3 Äquivalente des Salzes, bezogen auf den Ausgangsstoff, verwendet. Das Salz der schwefligen Säure kann in fester Form oder technisch vorteilhaft als gesättigte wäßrige Lösung eingesetzt werden. Die Wassermenge ist nicht besonders kritisch ,zweckmäßigerweise wird man einen möglichst geringen Anteil an Wasser wählen. Im allgemeinen können 0,5 bis 50, vorzugsweise 1 bis 5 Gew.-Teile Wasser je Teil 2,4-Dinitrochlorbenzol verwendet werden.

Zur Beschleunigung der Reaktion wird dem Reaktionsgemisch ein mit Wasser mischbares inertes Lösungs- oder Verdünnungsmittel (Cosolvens) zugesetzt, das eine Auflösung oder zumindest Anlösung des 2,4-Dinitrochlorbenzols bewirkt. Das Gewichtsverhältnis von Wasser zu Cosolvens liegt im allgemeinen bei 1:0,08 bis 1:2, vorzugsweise 1:0,6 bis 1,85. Als solches Lösungsmittel können beispielsweise niedermolekulare Carbonsäureamide wie N,N-Dimethylformamid oder Alkohole wie Methanol oder Ethanol dienen.

Im Hinblick auf die sich anschließende Hydrierung und die eventuelle Weiterverarbeitung der Metaminsäure ist es besonders vorteilhaft, den Chlor/Sulfitaustausch statt durch das Zufügen eines Cosolvens durch die Zugabe eines ionischen oder nichtionischen Tensids oder Dispergiermittels zu beschleunigen. Solche Hilfsmittel sind beispielsweise in Brockhaus, ABC Chemie, Bd. 1, Seite 503, 1971, VEB F.A. Brockhaus Verlag Leipzig oder in Ullmanns Enzyklopädie d. techn. Chemie, 3. Aufl., Bd. 16, Seiten 724 bis 735 bzw. 4. Aufl., Bd. 22, Seiten 455 bis 515 beschrieben. Es können anionaktive Tenside wie Sulfobernsteinsäureester (z.B. das Natriumsalz des Sulfobernsteinsäurebis(2-ethylhexyl)esters) oder Kondensationsprodukte von Naphthalinsulfonsäure mit Formaldehyd (Ullmann, 4. Aufl., Bd. 22, Seite 476, s.o.), kationaktive Tenside wie quartäre Ammoniumsalze mit 1 bis 3 Methyl-Resten und 3 bis 1 C₈ bis C₁₆-Alkylresten (z.B. Methyltrioctylammoniumchlorid, Trimethylpentadecylammoniummethosulfat) sowie vorzugsweise nichtionogene Tenside wie Ethylenoxidaddukte verwendet werden. Als Vertreter für letztere seien Ester oder Ether von Acylpolyethylenglykolen wie hochoxethylierte Fettsäuren mit 40 bis 60 Ethylenoxid-Einheiten (z.B. oxethyliertes Ricinusöl mit vorzugsweise 48 Ethylenoxid-Einheiten und oxethyliertes Oleylamin mit vorzugsweise 11 bis Ethylenoxid-Einheiten) aufgeführt.

Die genannten Hilfsstoffe werden im allgemeinen in katalytischer Menge, z.B. in einer Menge von 0,1 bis 20, vorzugsweise 1 bis 5, besonders bevorzugt 1 bis 2 Gew.%, bezogen auf 2,4-Dinitrochlorbenzol, verwendet.

Der pH-Wert des Reaktionsgemisches wird vor Zugabe des Sulfits oder Hydrogensulfits auf 6 bis 8, vorzugsweise 7 eingestellt, indem man beispielsweise ein anorganisches Hydroxid hinzugibt.

Der Chlor/Sulfitaustausch kann vorteilhaft bei einer Temperatur von 20 bis 100°C, vorzugsweise 50 bis 70°C, drucklos oder unter Druck, kontinuierlich oder diskontinuierlich nach den dafür üblichen Techniken durchgeführt werden.

Nach beendeter Umsetzung wird das Reaktionsgemisch angesäuert und auf einen pH von ca. 1 bis 5, vorzugsweise 3, gebracht, wodurch gegebenenfalls im Überschuß vorhandene Sulfitionen zerstört werden. Als Säure kommen anorganische oder organische Säuren in reiner Form oder als wäßrige Lösung in Betracht. So können beispielsweise Flußsäure, Salzsäure, Bromwasserstoffsäure, Jodwasserstoffsäure, Schwefelsäure, Salpetersäure, ortho-Phosphorsäure, Ameisensäure, Essigsäure, Citronensäure, Oxalsäure oder Weinsäure eingesetzt werden. Bevorzugt wird Schwefelsäure. Das sich bei der Ansäuerung bildende Schwefeldioxid kann technisch vorteilhaft durch Durchleiten eines inerten Gasstromes, vorzugsweise eine Stickstoffstromes, weitgehend aus dem Reaktionsgemisch entfernt werden. Vor der nachfolgenden Hydrierung kann es zweckmäßig sein, gegebenenfalls bei der Umsetzung gebildete, in Wasser unlösliche Bestandteile durch Filtration zu entfernen.

Die katalytische Hydrierung der 2,4-Dinitrobenzolsulfonsäure kann in an sich bekannter Weise in Gegenwart eines Promotors durchgeführt werden. Als Hydrierkatalysatoren kommen Metalle wie z.B. Nickel, Palladium, Platin, Rhodium, Ruthenium, Kobalt und Kupfer in Betracht. Vorzugsweise werden Raney-Nickel- und Palladium-Katalysatoren eingesetzt. Diese Katalysatoren können sowohl in Form des Metalls als auch auf einem Träger wie z.B. Kohlenstoff, Bariumsulfat, Silicagel oder Aluminiumoxid aufgebracht vorliegen. Nickel, Kobalt und Kupfer lassen sich auch als Raney-Katalysatoren einsetzen. Die Menge des Katalysators liegt im allgemeinen im Bereich von 0,01 bis 30 Gew.%, bezogen auf 2,4-Dinitrochlorbenzol als Metall, wobei ein Bereich von 2 bis 20, vorzugsweise 5 bis 10 Gew.% für den Raney-Katalysator und ein Bereich von 0,05 bis 5, vorzugsweise 0,05 bis 0,5 Gew.% (berechnet reines Metall) für den auf einem Träger aufgebrachten Katalysator vorteilhaft ist.

Die Hydrierung wird bei einem Druck von 1 bis 9, insbesondere 1 bis 6 bar kontinuierlich oder diskontinuierlich durchgeführt. Als Reaktionstemperatur kann ein Bereich von 0 bis 200°C, vorzugsweise 20 bis 90°C gewählt werden.

Der pH-Wert des Reaktionsgemisches sollte vorteilhaft zu Beginn der Hydrierung zwischen 3 und 7, vorzugsweise bei 4 bis 6 liegen.

Als Promotoren kommen eine anorganische oder organische Säure oder das Salz eines in verschiedenen Oxidationsstufen stabilen Metalls in Frage. Als Säure können z.B. Borsäure, Phosphorsäure, Ameisensäure, Essigsäure, Propionsäure, Oxalsäure, Malonsäure, Bernsteinsäure, Maleinsäure, Benzoesäure, Phthalsäure, p-Toluolsulfonsäure und Benzolsulfinsäure sowie Gemische dieser Säuren eingesetzt werden. Bevorzugt sind Propionsäure, Phosphorsäure und Borsäure. Als Salze kommen z.B. Kupfer(I)chlorid, Eisen(II)chlorid und Zinn(II)chlorid in Frage. Bevorzugt wird Eisen(II)chlorid. Die organischen Säuren können in einer Menge von 0,1 bis 3, vorzugsweise 1 bis 2 Äquivalenten, die anorganischen Säuren in einer Menge von 0,01 bis 2, vorzugsweise 0,01 bis 1 Äquivalenten und die Salze in einer Menge von 0,001 bis 1, vorzugsweise 0,002 bis 0,5 Äquivalenten, jeweils bezogen auf 2,4-Dinitrochlorbenzol eingesetzt werden.

Nach beendeter Wasserstoffaufnahme kann das Reaktionsgemisch in üblicher Weise aufgearbeitet werden, z.B. indem man den Hydrierkatalysator abfiltriert und die Metaminsäure durch Ansäuren oder Aussalzen aus der wäßrigen Lösung gewinnt. In der Regel ist eine Isolierung der Metaminsäure jedoch nicht nötig, da die im Filtrat vorliegende Metaminsäure direkt weiterverarbeitet werden kann, z.B. zu Reaktivfarbstoffen (DE-PS 95 97 48 und 10 47 339).

Nach den erfindungsgemäßen Verfahren ist es überraschend möglich, Metaminsäure in technisch einfacher Weise ohne Isolierung von Zwischenstufen in hohen Ausbeuten herzustellen.

### Beispiel 1

286 g 2,4-Dinitrochlorbenzol wurden in 1000 ml Wasser angerührt und mit 6,4 g 50%iger Natronlauge sowie 4 g eines Oleylaminpolyethylenglykolethers mit 11 bis 12 Ethylenoxideinheiten versetzt. Dazu tropfte man bei 60°C innerhalb von 90 min. eine Lösung von 218 g Natriumsulfit in 700 ml Wasser und ließ 2 Stunden lang bei dieser Temperatur nachrühren. Anschließend säuerte man die Reaktionslösung mit 1,5 g 50%iger Schwefelsäure an, leitete 3 Stunden lang Stickstoff durch, filtrierte die unlöslichen Bestandteile ab, gab zum Filtrat 27,8 g 50%ige Natronlauge, 111 g Propionsäure sowie 30 g einer 57%igen wäßrigen Suspension von Raney-Nickel und hydrierte 6 Stunden lang bei 43°C und einem Wasserstoffdruck von 3,0 bar. Danach wurde der Katalysator abfiltriert und mit Wasser gewaschen. Die vereinigte Filtrate stellten 2860 g einer 8,9%igen wäßrigen Metaminsäurelösung ≙ 255 g (96 % d.Th.) Wertprodukt, dar.

### Beispiel 2

Führte man den Sulfitaustausch und die Hydrierung wie in Beispiel 1 beschrieben durch und ersetzt die Propionsäure durch ein Gemisch aus 3,7 g 85%iger Phosphorsäure und 70 g Borsäure, so erhielt man 2780 g einer 9,0%igen wäßrigen Metaminsäurelösung ≙ 250 g (94 % d.Th.) Wertprodukt.

### Beispiel 3

Man führte den Sulfitaustausch wie in Beispiel 1 beschrieben durch und gab nach dem Abfiltrieren der unlöslichen Bestandteile 3,9 Teile eines Katalysators mit 5 % Palladium auf Kohle sowie 0,8 Teile Eisen(II)-chlorid-tetrahydrat zu. Dieses Gemisch wurde 6 Stunden lang bei 90°C und einem Wasserstoffdruck von 3,0 bar hydriert. Nach dem Abfiltrieren des Katalysators verblieben 2650 g einer 9,5%igen wäßrigen Metaminsäurelösung ≙ 252 g (95 % d.Th.) Wertprodukt.

## Patentansprüche

1. Verfahren zur Herstellung von Metaminsäure, dadurch gekennzeichnet, daß man in einer ersten Stufe 2,4-Dinitrochlorbenzol in wäßrigem Medium in Gegenwart
a) eines organischen Lösungs- oder Verdünnungsmittels und/oder
b) katalytischer Mengen eines ionischen oder nichtionischen Tensids oder Dispergiermittels
bei einem pH von 6 bis 8 mit Salzen der schwefligen Säure umsetzt, das Reaktionsgemisch ansäuert und die so gebildete 2,4-Dinitrobenzolsulfonsäure in situ katalytisch bei Atmosphärendruck oder Drücken von 1 bis 9 bar in Gegenwart eines Promotors hydriert.

2. Verfahren nach Anspruch 1, dadurch gekennzeichnet, daß man als Tensid oder Dispergiermittel Ether oder Ester von Acylpolyethylenglykolen verwendet.

3. Verfahren nach den Ansprüchen 1 und 2, dadurch gekennzeichnet, daß man als Tensid oder Dispergiermittel einen Fettaminpolyethylenglykolether verwendet.

4. Verfahren nach den Ansprüchen 1 bis 3, dadurch gekennzeichnet, daß man 0,1 bis 20 Gew.% des Tensids oder Dispergiermittels bezogen auf 2,4-Dinitrochlorbenzol verwendet.

5. Verfahren nach Anspruch 1, dadurch gekennzeichnet, daß man pro Mol 2,4-Dinitrochlorbenzol 1 bis 2 Äquivalente eines Salzes der schwefligen Säure verwendet.

6. Verfahren nach Anspruch 1, dadurch gekennzeichnet, daß man als Katalysator Raney-Nickel oder Palladium verwendet.

7. Verfahren nach Anspruch 1, dadurch gekennzeichnet, daß man die Hydrierung bei einem Druck von 1 bis 6 bar vornimmt.

8. Verfahren nach Anspruch 1, dadurch gekennzeichnet, daß man als Promotor Propionsäure, Phosphorsäure oder Borsäure verwendet.

9. Verfahren nach Anspruch 1, dadurch gekennzeichnet, daß man als Promotor Kupfer(I)chlorid, Eisen(II)chlorid oder Zinn(II)chlorid verwendet.

## Claims

1. A process for preparing 2,4-dinitrobenzenesulfonic acid, which comprises reacting in a first stage 2,4-dinitrochlorobenzene in an aqueous medium with salts of sulfurous acid in the presence of
a) an organic solvent or diluent and/or
b) catalytic amounts of an ionic or nonionic surfactant or dispersant
at a pH of from 6 to 8, acidifying the reaction mixture, and catalytically hydrogenating the resulting 2,4-dinitrobenzenesulfonic acid in situ at atmospheric pressure or at pressures of from 1 to 9 bar in the presence of a promoter.

2. A process as claimed in claim 1, wherein the surfactant or dispersant used is an ether or ester of an acylpolyethylene glycol.

3. A process as claimed in claim 1 or 2, wherein the surfactant or dispersant used is a fatty amine polyethylene glycol ether.

4. A process as claimed in any of claims 1 to 3, wherein the surfactant or dispersant is used in an amount of from 0.1 to 20% by weight, based on 2,4-dinitrochlorobenzene.

5. A process as claimed in claim 1, wherein from 1 to 2 equivalents of a salt of sulfurous acid are used per mole of 2,4-dinitrochlorobenzene.

6. A process as claimed in claim 1, wherein the catalyst used is Raney nickel or palladium.

7. A process as claimed in claim 1, wherein the hydrogenation is carried out at a pressure of from 1 to 6 bar.

8. A process as claimed in claim 1, wherein the promoter used is propionic acid, phosphoric acid or boric acid.

9. A process as claimed in claim 1, wherein the promoter used is copper(I) chloride, iron(II) chloride or tin(II) chloride.

## Revendications

1. Procédé de préparation d'acide 2,4-diaminobenzènesulfonique, caractérisé en ce que, dans une première étape, on fait réagir du 2,4-dinitrochlorobenzène avec des sels de l'acide sulfureux en milieu aqueux, en présence
a) d'un solvant ou diluant organique et/ou
b) de quantités catalytiques d'un tensio-actif ou agent dispersant ionique ou non ionique,
à un pH de 6 à 8, on acidifie le mélange réactionnel et on hydrogène catalytiquement in situ l'acide 2,4-dinitrobenzènesulfonique ainsi formé, sous la pression atmosphérique ou sous des pressions de 1 à 9 bar, en présence d'un promoteur.

2. Procédé selon la revendication 1, caractérisé en ce qu'on utilise, comme tensio-actifs ou agents dispersants, des éthers ou des esters de polyéthylèneglycols acylés.

3. Procédé selon la revendication 1 ou 2, caractérisé en ce qu'on utilise, comme tensio-actif ou agent dispersant, un oxyde d' amine grasse et de polyéthylèneglycol.

4. Procédé selon l'une quelconque des revendications 1 à 3, caractérisé en ce qu'on utilise de 0,1 à 20% en poids du tensio-actif ou agent dispersant par rapport au 2,4-dinitrochlorobenzène.

5. Procédé selon la revendication 1, caractérisé en ce qu'on utilise, par mole de 2,4-dinitrochlorobenzène, 1 à 2 équivalents d'un sel de l'acide sulfureux.

6. Procédé selon la revendication 1, caractérisé en ce qu'on utilise, comme catalyseur, du nickel de Raney ou du palladium.

7. Procédé selon la revendication 1, caractérisé en ce qu'on conduit l'hydrogénation sous une pression de 1 à 6 bar.

8. Procédé selon la revendication 1, caractérisé en ce qu'on utilise, comme promoteur, de l'acide propionique, de l'acide phosphorique ou de l'acide borique.

9. Procédé selon la revendication 1, caractérisé en ce qu'on utilise, comme promoteur, du chlorure cuivreux, du chlorure ferreux ou du chlorure stanneux.
